# EUROPEAN PATENT SPECIFICATION

(11) **EP 1 531 728 B1**
(45) Date of publication and mention of the grant of the patent: **28.07.2010**
(21) Application number: 03733722.7
(22) Date of filing: 11.06.2003
(51) Int. Cl.: A61B 6/03, A61B 8/14, A61B 6/08

(54) **PATIENT REPRESENTATION IN MEDICAL MACHINES**
PATIENTENREPRÄSENTATION IN MEDIZINISCHEN GERÄTEN
REPRESENTATION DU PATIENT DANS DES MACHINES MEDICALES

(30) Priority: 24.06.2002 US 176650
(43) Date of publication of application: 25.05.2005
(60) Divisional application: 10163076.2
(73) Proprietor: C-Rad Positioning AB, 753 23 Uppsala (SE)
(72) Inventor: SJÖGREN, Bo, S-737 30 Fagersta (SE); SKATT, Björn, S-142 63 Trångsund (SE); BRAHME, Anders, S-182 33 Danderyd (SE); LÖF, Johan, S-182 34 Danderyd (SE); COBLENTZ, Pedro, S-171 61 Solna (SE); JANSSON, Allan, S-187 34 Täby (SE)
(74) Representative: Stenborg, Anders Vilhelm
(86) International application number: PCT/SE2003/000964
(87) International publication number: WO 2004/000120

(56) References cited:
- WO-A1-02/19908
- US-A- 5 447 154
- US-A- 5 727 554
- US-A- 5 999 840
- US-A- 6 032 066
- US-A- 6 151 404
- US-B1- 6 325 758

## Description

### TECHNICAL FIELD OF THE INVENTION

The present invention generally relates to the management of anatomical patient information in therapeutic and/or diagnostic processes, and in particular to methods and systems for accurate patient positioning and for coordinating anatomical patient information in such processes.

### BACKGROUND OF THE INVENTION

During the past decades there have been considerable developments within the fields of radiation therapy and medical diagnosis. The performance of external beam radiation therapy accelerators, brachytherapy and other specialized radiation therapy equipment has improved rapidly. Developments taking place in the quality and adaptability of radiation beams have included new targets and filters, improved accelerators, increased flexibility in beam-shaping through new applicators, collimator and scanning systems and beam compensation techniques, and improved dosimetric and geometric treatment verification methods have been introduced.

Furthermore, a number of powerful 3-dimensional diagnostic techniques have been developed, ranging from computed tomography (CT), positron and single photon emission computed tomography (PET and SPECT) to ultrasound and magnetic resonance imaging and spectroscopy (MRI and MRS). Equally important is the increased knowledge of the biological effect of fractionated uniform and non-uniform dose delivery to tumors and normal tissues and new assay techniques, including the determination of effective cell doubling times and individual tissue sensitivities, allowing optimization of the dose delivery to tumors of complex shape and advanced stages.

However, one of the weakest links in this development in radiation therapy treatment has been the process of relating measured anatomical patient information, including position and shape of the tumor and adjacent tissues and organs, between diagnostic machines and the actual radiation therapy machine. This is a twofold problem originating both from inaccuracies in patient positioning in the medical machines and difficulties in coordinating anatomical information from different diagnostic machines and techniques.

An accurate positioning of the patient in the diagnostic machines of the radiation therapy or diagnostic process and above all during the radiation treatment in the radiation therapy machine is vital for an effective and accurate treatment. The position and shape of internal tissues and organs, including the tumor, depends on the actual position and posture of the patient, with possible spatial differences of organ positions of tens of millimeters. The most widely used positioning technique today is the isocentric method. In the treatment room, lasers producing laser beams are arranged. The beams cross exactly at the isocenter or the origin of the room coordinate system. When the patient is placed on the couch, the isocenter is inside the body, thus the laser beams can be seen as bright dots on the surface of the skin. During a treatment simulation, the patient is positioned as accurately as possible with e.g. diagnostic X-ray or portal imaging. Once the correct patient position is obtained, the positions of the bright dots are marked with special ink, which stays in the skin for weeks. The next time the patient is to be positioned, it is sufficient to align the marks with the laser beams. However, a major problem is that since the skin is not rigidly connected to the bony structures, the skin moves and stretches depending on how the patient lies, i.e. the patient's posture. The skin may also change shape through weight loss or swelling during the time of treatment. Therefore, this method typically gives an error of 5 to 8 millimeters, with occasional outliers of 10 millimeters or more.

US Patent 5,080,100 discloses a method and device for verification of the precise position of a patient in a radiation therapy machine. A device is mounted on the movable arm of a mount with isocentric motion. This device includes a system for scanning by a light beam. The position of the source of this light beam corresponds to the position of the radiation source. The device further has a system for optical detection of the point of impact of the light beam on the patient. These two systems enable the position of the point of impact to be determined by means of a data-processing system.

In addition, the position and posture of the patient may change during the treatment due to movement of the patient, filling of the bladder etc. Such a repositioning may cause the radiation beams to ineffectively hit the target volume, or completely miss it and instead hit adjacent tissues and organs. In US Patent 5,727,554 a camera generates digital image signals representing an image of one or more natural or artificial fiducials on a patient positioned on a treatment or diagnosis machine. A processor applies multiple levels of filtering at multiple levels of resolution to repetitively determine successive fiducials positions. A warning signal is generated if movement exceeds certain limits but is still acceptable for treatment. An unacceptable displacement results in termination of the treatment beam.

In the diagnosis and treatment process or treatment planning procedure, anatomical information from several different diagnostic machines, e.g. a CT and MRI machine, is used to get a complete and detailed picture of the target volume with the tumor and adjacent organs and tissues, through which the beams pass to hit the target volume. Today, no satisfactory method exists to integrate and coordinate the anatomical information obtained using these different diagnostic techniques. Instead, the medical personnel either manually or by means of computers tries to visually match common structures and reference points found in the information from the machines. This is a tedious and highly inefficient procedure, the accuracy of which almost exclusively depends on the judgment of the personnel.

US 6,325,758 discloses verification of the position of a target within a patient body to be treated by a radiation therapy machine. A CT or MR machine is used to generate anatomical data of the patient that is used in the generation of a treatment plan, which includes at least two 2D patient images of the target to be irradiated. In the radiation therapy machine an ultrasound probe is produce at least two 2-dimentsional ultrasound images of the target. Thereafter, the two ultrasound images and the two patient representations from the treatment plan are displayed. The displayed target representations are aligned with the displayed ultrasound images and the amount and type of movement of the treatment table, patient and/or the radiation therapy machine required to dispose the target, with respect to the ultrasound images and radiation therapy machine, to conform to the desired position of the target in the radiation treatment plan is determined.

US 5,447,154 discloses the determination of the position of a patient's organ with respect to at least two imaging devices. In a diagnostic machine having an associated first coordinate system, a 3D functional anatomical image is generated, which image also illustrates a surface of the depicted organ or a skin surface. The patient is then subsequently positioned on a treatment table with a second coordinate system. The position of a 3D morphological imaging machine arranged in connection with the treatment table is determined in the second coordinate system. This imaging machine is used for generating a 3D morphological surface image of the patient's organ or skin. The 3D functional image is then matched with the 3D morphological image. A third medical machine, e.g. second diagnostic machine or a radiation therapy machine, is also arranged together with the treatment table and the imaging machine. This third medical machine is associated with a third coordinate system. The relative positions of this third medical machine and the imaging machine are determined. Using this determined relative position, the third coordinate system may be positioned with respect to the 3D morphological image of the imaging machine. Furthermore, due to the matching between the 3D morphological image and the 3D functional image, the third coordinate system may be positioned with respect to the 3D functional image.

US 5,999,840 discloses registration of 3D surgical image data utilized in image guided surgery. 3D anatomical data is collected by MRI or CT of a patient portion in a first coordinate system. The patient is then moved to the operation room, which is equipped with a laser scanning system and a real-time video camera. The laser scanning system is used for collecting a 3D surface image of the patient portion in a second coordinate system. A complex image matching process is performed based on the 3D anatomical data and the 3D surface image for the purpose of determining a transform between the first and second coordinate systems. The laser scanning system and the real-time video camera are positioned to detect the same landmarks and therefore allow a transform between the second coordinate system and a third coordinate system of the video camera. The determined transforms can be used to realign the 3D anatomical data into the same plane as the video image. Also a mixing of 3D anatomical data and live video images is possible.

### SUMMARY OF THE INVENTION

The present invention overcomes these and other drawbacks of the prior art arrangements.

It is a general object of the invention to improve the accuracy of radiation therapy and diagnostic processes.

It is also an object of the invention to provide a method and system for relating anatomical patient information between different medical machines.

Yet another object of the invention is to provide a method and system for accurate patient positioning in medical machines.

A further object of the invention is to provide a method and system being able to accurately and effectively integrate and coordinate anatomical patient information from different diagnostic machines.

These and other objects are met by the invention as defined by the accompanying patent claims.

Briefly, the general concept of the present invention is to determine, in connection with each of a number of medical machines or equipment, including diagnostic and radiation therapy machines, a 2- or 3-dimensional representation of at least a portion of a patient in relation to an overall common coordinate system associated with the patient. Based on the determined 2- or 3-dimensional representations, anatomical patient information may be related between the different machines. In other words, since the 2- or 3-dimensional representations are determined in the overall coordinate system and a transformation between the representations and the anatomical information can be determined, the 2- or 3-dimensional representations are used as a common reference between the medical machines in order to facilitate and make it possible to relate and possible also depict and delineate anatomical information between different medical machines in the common coordinate system.

The 2- or 3-dimensional patient representations are preferably determined using a laser scanning system to obtain surface profile measurements of the patient based on the laser scanning. More preferably, the patient representation is a 3-dimensional surface representation of at least a portion of the skin of the patient.

In a typical scenario, anatomical information from a first medical machine is matched with a 2- or 3-dimensional representation measured in the first medical machine. The 2- or 3-dimensional representation of the first medical machine is then matched with a corresponding 2- or 3-dimensional representation from a second medical machine in order to relate the anatomical information from the first machine for use in the second machine.

Suitable applications in the radiation therapy or diagnostic process may be accurate positioning of the patient and for coordinating anatomical patient information from different diagnostic machines.

For positioning purposes, a reference representation of the patient is determined based on a 2- or 3-representation of the patient in a first medical machine. When the patient subsequently is positioned in a second medical machine, a likewise 2- or 3-representation of the patient is determined and compared to the reference representation. The difference, or deviation, between the 2- or 3-dimensional representations is normally used to adjust the position of patient, either automatically or manually based on e.g. a depicted illustration of the deviation. In a preferred implementation, a control signal is determined based on the deviation and is used for automatic adjustment of a patient couch. The accurate patient positioning obtained with the present invention, is highly advantageous for diagnostic and radiation therapy processes, both enabling anatomical information from different diagnostic machines to be integrated in the treatment planning and efficient and safe treatment in the radiation therapy machine. This embodiment of the invention can also be used for monitoring patient position during the actual treatment, where a large misplacement or movement of the patient may result in abortion of the treatment, in turn contributing to the safe and efficient treatment.

The present invention may also be used for coordinating anatomical patient information, obtained from different medical machines, e.g. computed tomography (CT) and magnetic resonance (MR) machines. The anatomical information from the different diagnostic machines is integrated into the common coordinate system based on the respective 2- or 3-representations as common reference between the machines.

In a preferred embodiment of the invention different transformations are used to transform coordinate data from the local coordinate system of the medical machines and the coordinate system associated with the 2- or 3-dimensional measuring system to the overall coordinate system.

The invention offers the following advantages:
- Increased accuracy in patient positioning in a radiation therapy or diagnostic process;
- Continuous monitoring of patient position;
- Automated and simple process of integrating and depicting anatomical patient information from different imaging modalities and techniques; and
- Can be used with different types of diagnostic and radiation therapy machines and equipment.

Other advantages offered by the present invention will be appreciated upon reading of the below description of the embodiments of the invention.

### BRIEF DESCRIPTION OF THE DRAWINGS

The invention, together with further objects and advantages thereof, will be best understood by reference to the following description taken together with the accompanying drawings, in which:
Fig. 1 is a schematic drawing of a general radiation therapy process according to the invention;
Fig. 2 is a more detailed representation of a (the) general radiation therapy process;
Fig. 3 illustrates schematically a radiation therapy machine incorporating a patient representation measuring system according to the present invention;
Figs. 4A-D illustrate general principles of obtaining a 3-dimensional surface representation with a photon-based representation system;
Fig. 5 is a schematic block diagram of a system for relating anatomical patient information between different medical machines according to the invention;
Fig. 6 is a drawing illustrating a comparison between two 3-dimensional surface representations obtained according to the present invention; and
Figs. 7A-D illustrate the process of integrating and coordinating anatomical information according to the invention.

### DETAILED DESCRIPTION OF EMBODIMENTS OF THE INVENTION

Throughout the drawings, the same reference characters will be used for corresponding or similar elements.

Although the expression 'medical machine or equipment' normally relates to diagnostic machines and radiation therapy machines in the present description, it should be understood that the invention is generally applicable to different medical machines, such as surgery equipment, e.g. robotic surgery appliance. Diagnostic machines or imaging machines are used to obtain anatomical information of a patient, including localization of tumors and adjacent tissues and organs, based on different imaging techniques. Such imaging techniques may be e.g. computed tomography (CT), including conventional CT and cone-beam CT imaging, radiation therapy CT (RCT) and other diagnostic X-ray techniques, positron emission computed tomography (PET), single photon emission computed tomography (SPECT), combined PET and CT (PET/CT), ultrasound, magnetic resonance (MR) techniques, e.g. magnetic resonance imaging (MRI) and magnetic resonance spectroscopy (MRS) and other imaging techniques. Based on the obtained anatomical information, radiation treatment may be performed in a radiation therapy machine, where a dose package or radiation beam, such as a beam of gamma photons, electrons, neutrons, protons or heavier ions, atoms or molecules, is applied to a patient, possibly including non-human animal patients. The radiation therapy machine may be employed for curative radiation therapy, i.e. to eradicate a tumor, or palliative radiation therapy, where the aim is generally to improve quality of life of the patient by maintaining local tumor control, relieve a symptom or prevent or delay an impending symptom, and not primarily to eradicate the tumor. Yet another application of a radiation therapy machine may be in radiosurgery using a high-energy radiation source. The process of using different diagnostic imaging machines and techniques to obtain anatomical information of a patient is in the present description denoted a diagnostic process. In a radiation therapy process, radiation therapy treatment is performed based on obtained anatomical patient information. This means that the overall radiation therapy process includes all the steps from diagnosing to the actual radiation therapy treatment and follow-up and evaluation procedures, and thus normally includes a diagnostic process.

In the present description the expression 'relating anatomical patient information between different medical machines', generally means the process of using anatomical patient information from a first medical machine in a second medical machine. The information may be used in the operation of the second medical machine, for accurate positioning of a patient in the machine, for coordinating or aligning the anatomical information with similar patient information obtained in the second medical machine etc. 'Coordinating anatomical patient information from different medical (diagnostic) machines' is referred to the process of integrating or aligning anatomical patient information obtained from the medical machines such that the information may be used, e.g. depicted or delineated, together in a common overall coordinate system. However, the information could also or instead be integrated together in the form of a data set of the coordinates of respective patient information in the overall coordinate system, i.e. without any actual visualization of the information.

Briefly, the general concept of the present invention is to determine, in connection with each of a number of medical machines, including diagnostic and radiation therapy machines, a 2- or 3-dimensional representation of at least a portion of a patient in relation to an overall common coordinate system associated with the patient. Based on the determined 2- or 3-dimensional representations, anatomical patient information may be related between the different machines. In other words, since the 2- or 3-dimensional representations are determined in the overall coordinate system and a transformation between the representations and the anatomical information can be determined, the 2- or 3-dimensional representations are used as a common reference between the medical machines in order to facilitate and make it possible to relate and possible also depict and delineate anatomical information between different medical machines in the common coordinate system.

Suitable applications in the radiation therapy or diagnostic process may be in accurate positioning of the patient and for coordinating and integrating anatomical patient information from different diagnostic machines.

For positioning purposes, a reference representation of the patient is determined based on a 2- or 3-dimensional representation of the patient in a first medical machine. When the patient subsequently is positioned in a second medical machine, a likewise 2- or 3-dimensional patient representation is determined and compared to the reference representation. The position of the patient may then be adjusted until the deviation between the measured representation in the second medical machine and the reference representation is below a given threshold.

In coordinating anatomical patient information, the anatomical information from different diagnostic machines are integrated and possibly depicted or delineated into the common coordinate system based on the respective 2- or 3-representations as common reference between the machines.

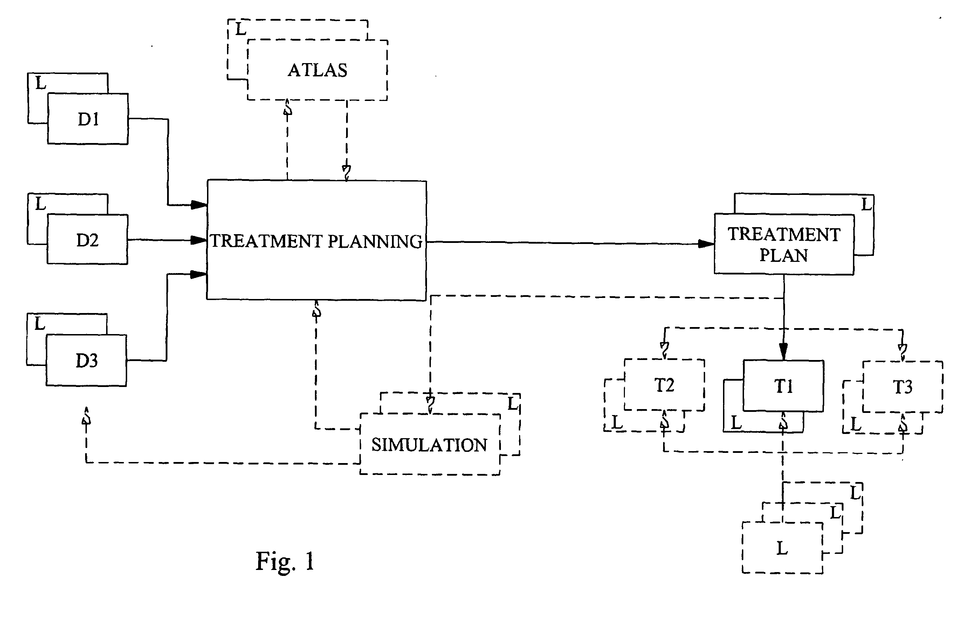

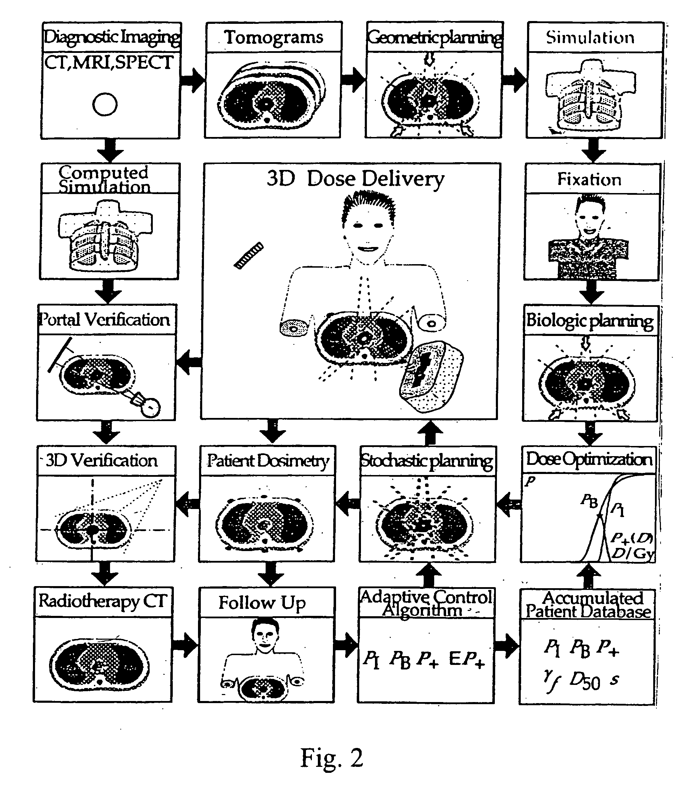

For a better understanding of the invention, it may be useful to start with a brief introduction of the radiation therapy process with reference to Figs. 1 and 2.

Generally, the first step in a radiation therapy process is performing a diagnostic process or diagnosing. Different diagnostic machines are employed to localize a tumor and adjacent tissues and organs. This diagnostic anatomical information D1, D2, D3 is used to as accurately as possible pinpoint the exact location of the tumor in the patient and detect any organs or tissues that may be affected or should be avoided by the radiation beam in the subsequent radiation therapy treatment. It is normally advisable to use anatomical information D1, D2, D3 from different diagnostic machines, since different imaging techniques give different anatomical information. For an example, CT is superior for obtaining density information and MRI for retrieving anatomical information about soft tissues near bony structures, such as the central nervous system. Therefore, information D1, D2, D3 from different diagnostic machines complement each other and should together give a sufficient picture of the target volume and surrounding tissues.

Based on the measured anatomical information, a treatment or dose planning process is carried out. In the treatment planning the goals are generally to:
- Achieve the desired dose in the target volume;
- Uniformly distribute the dose in the target volume;
- Avoid high doses in surrounding tissues and organs and in organs at risk; and
- Limit the total dose received by the patient.

To achieve these goals, the measured anatomical information is investigated to define the target volume and identify organs at risk. Thereafter, dose prescription for the target volume and tolerance level of organs at risk are specified. Further, radiation modality and treatment technique are selected for the particular treatment. Having decided treatment technique the number of beam portals (sources) and the directions of the incidence of the beams are selected and optimized, considering the present anatomical information. Also beam collimation, beam intensity profiles, fractionation schedule, etc. are selected and optimized based on the actual patient information. Once these parameters are optimized, a dose distribution in the patient is calculated and, if it fulfils the general goals, a treatment or dose plan is composed.

The treatment plan should include all relevant information for the actual radiation therapy treatment, such as the selected and optimized parameters from the treatment planning and the present set-up of the radiation therapy machine and its settings. Before the actual radiation therapy treatment an optional treatment simulation may be performed to test and verify the treatment plan. In the simulation procedure, the settings and equipment according to the treatment plan are used. Often portal images, i.e. images based on the treatment beam itself, are used to verify the treatment and monitor its reproducibility. Furthermore, e.g. *in vivo* dosimetry or related techniques may be used to check the delivered radiation dose in the target volume and/or in adjacent tissues, preferably in organs at risk. If the measured data corresponds to the calculated data in the treatment plan, the actual radiation therapy treatment may be initiated. However, if some divergence between the measured and calculated data is detected and the divergence exceeds a safety threshold, a change in the treatment plan must be performed. This change may in some cases simply be a resetting of parameters but also a larger change in the treatment plan, such as completing the treatment planning process with more anatomical information from a new diagnostic measurement. Either way, a new treatment plan is determined, which may be tested and verified in an optional new treatment simulation.

A radiation therapy treatment T1 is then performed with the equipment, set-up and settings specified in the treatment plan. It is vitally important that the patient is positioned accurately, based on the treatment plan, in the radiation therapy machine. A misplacement of only a few millimeters may cause damages to adjacent tissues and organs and make the treatment ineffective. Once the positioning is ready, the beams irradiate the patient according to the treatment plan to deliver the calculated dose in the target volume.

Although, the radiation therapy treatment in the section above has been described in relation to a single treatment occasion T1, the actual dose delivery is most often fractionated into several, often 20-30, fractions. This means that a total radiation therapy treatment usually extends over a period of days, weeks or in some occasions even months. After each treatment occasion, a follow-up or treatment monitoring evaluates the hitherto performed radiation therapy, possibly leading to changes in the treatment plan before the next treatment fraction, similar to the simulation procedure discussed above. In addition, different treatment machines may be employed, schematically illustrated by T1, T2 and T3 in Fig. 1. For example, at one treatment occasion a high-energy radiosurgery machine is used, whereas at the next occasion the treatment is performed with a radiation therapy machine adapted for curative radiation therapy. In this context, also medical machines not using curative, palliative or surgery radiation may be used. A typical example are different surgical equipment and appliances, where accurate patient positioning and/or coordinating anatomical patient information is required, such as equipment containing surgical robots.

As was briefly mentioned above, according to the invention, a 2- or 3-dimensional representation of the patient is determined in connection with a medical machine in relation to a common overall coordinate system. For a diagnostic machine, this means that the representation is measured in connection with the measurements of the anatomical patient information. In a radiation therapy machine, including treatment simulation machines, the 2- or 3-dimensional representation is measured before, during and/or after the actual dose delivery from the beam sources.

In a first embodiment of the invention, the 2- or 3-dimensional representation is a surface representation of the patient, e.g. a surface representation of the skin of the patient. In the case of a 2-dimensional representation, a single contour line somewhere on the body of the patient is measured. Due to the changing contour of the body when going in a longitudinal direction from one end to the opposite end, it is possible to measure a unique contour line almost everywhere over the body surface. However, to get a more accurate representation, a 3-dimensional surface representation may instead be used. In such a case, the whole body surface or a suitable selected portion thereof is measured to provide the 3-dimensional representation, where a larger surface often implies a more accurate representation. The surface representation may be a continuous 3-dimensional surface of a portion of the body or several dispersed surfaces, the relative spatial relationship of which is known. Preferred dispersed surfaces coincidence with some of the standard anatomical reference points used in radiation therapy. These points have only very little tissue over the underlying skeleton and are therefore rather stable even if the skin is stretched. The standard reference points comprise e.g. upper and distal edges of ilium, upper point of symphysis pubis, distal point of scapula, upper point of nose, upper and lower point of patella and lower point of fibula. However, the 2- or 3-dimensional surface representation may be measured on any suitable portion of the patient's body, and especially in close connection with the tumor, e.g. by measuring the body portion contour directly above the tumor position.

Instead of using a surface representation of the patient, other 2- or 3-dimensional anatomical representations of the patient may alternatively be used. In such a case, it is recommendable to use a 2- or 3-dimensional representation of at least a portion of the patient's skeleton. A 2-dimensional skeleton representation may a section of the patient's body showing a sectional portion of the skeleton. In a 3-dimensional representation, several such 2-dimensional sections may be combined to provide a 3-dimensional picture of a portion of the skeleton. As for the surface representations above, the skeleton representation may be measured on any suitable portion of the patient's body and especially comprising skeleton portions adjacent to the tumor tissue. If a more complete representation is preferred the representation may include the whole or a major portion of the patient's skeleton.

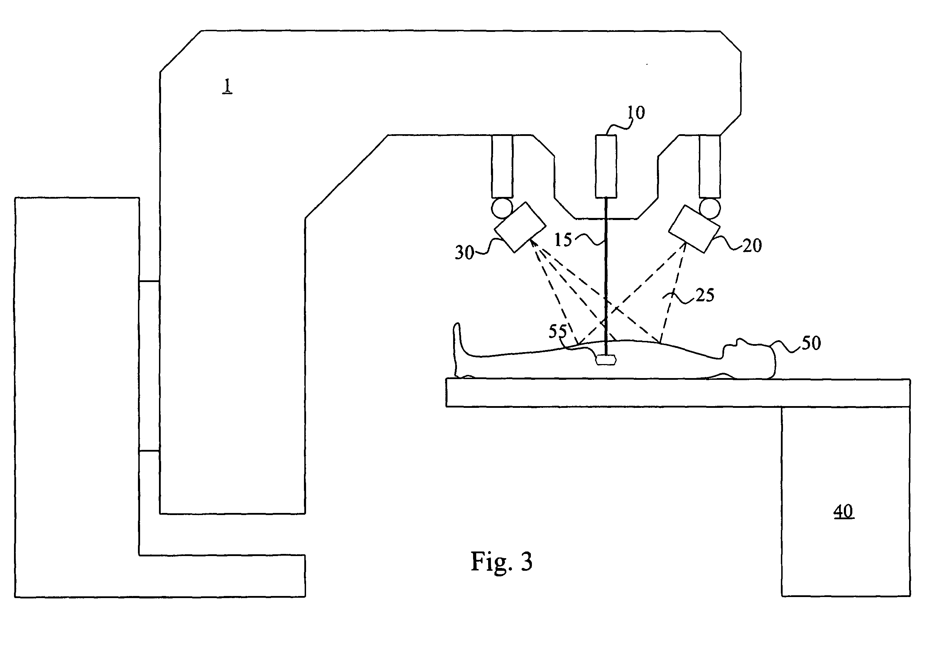

The 2- or 3-dimensional representations of the patient are measured in connection with different medical machines. An example of such a medical machine incorporating a system for patient representation measurement is schematically illustrated in Fig. 3, with the medical machine being represented by a radiation therapy machine 1. In Fig. 3, a patient 50 is positioned on a couch 40 and is irradiated by a treatment beam 15 from a radiation source 10 in the machine 1. A radiation target volume is schematically depicted as 55 in the drawing. In addition to this standard radiation machine, the radiation therapy machine 1 is provided with a 2- or 3-dimensional representation measuring system comprising a scanning device 20 and an image detector or receiver 30. The scanning device sends an imaging beam 25 onto the patient. The detector 30 then captures the beam and provides representation information. This beam may be a reflected beam from the patient as in Fig. 3, or a beam passing through the patient 50 and captured by a detector 30, arranged on the opposite side of the patient 50 relative to the scanning device 20. Based on the detected imaging data, the 2- or 3-dimensional representation is obtained. Although the scanning device 20 and the image detector 30 have been arranged onto the therapy machine 1 in Fig. 3, other arrangements are possible. For an example the scanning device 20 and/or the detector 30 may be arranged onto a dedicated frame, scaffold or rack, which is provided in the treatment room, in the vicinity of the radiation therapy machine 1. A similar arrangement is used for diagnostic machines, but then the radiation source 10 is exchanged with a diagnostic system, including a suitable transmitting source and an associated adapted detector.

In addition, the representation measuring system may include one scanning device and detector as in Fig. 3, but also several scanning devices and/or detectors arranged at different positions on and/or adjacent the medical machine. Using at least two scanning devices and detectors, a more accurate coverage of a larger portion of the patient without any missed areas and thus a better 2- or 3-dimensional representation is obtained.

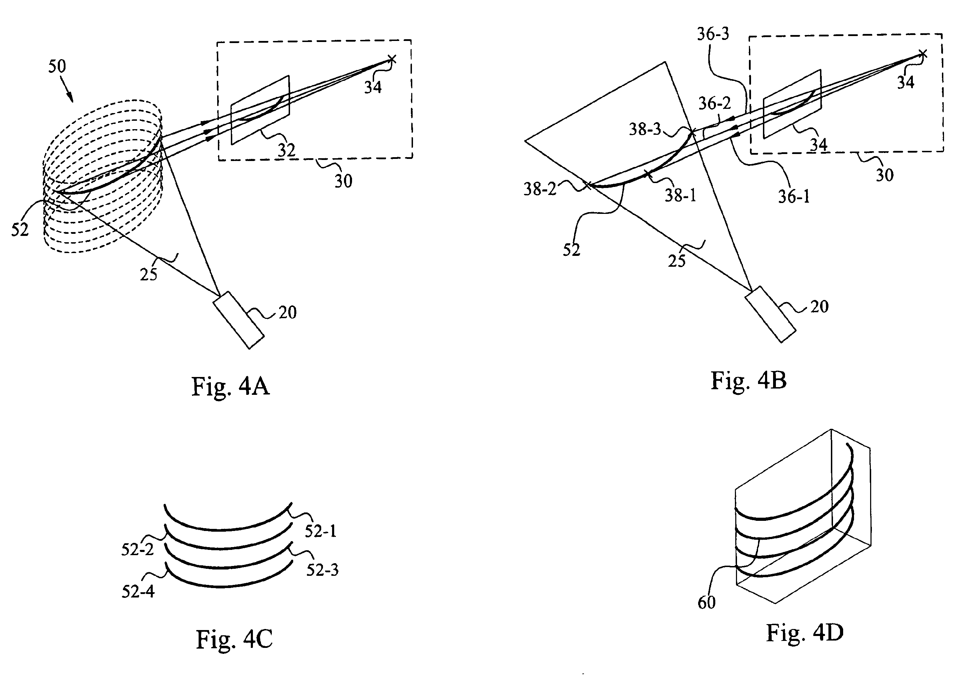

Suitable, but not limiting techniques used by the 2- or 3-dimensional representation measuring system in Fig. 3 include photon- or phonon-based techniques. Figs. 4A-D illustrate a photon-based technique in form of a laser scanning system. Starting with Fig. 4A, a laser scanning device 20 sends out a sheet of laser light 25 hitting a surface of a patient 50. A bright line 52 on the body 50 is reflected and detected by an image detector 30. The image detector 30 of Fig. 4A is schematically modeled as a focus 34 and a surface 32. In Fig. 4B, the measured image of the bright line 52 is used to reconstruct the body contour. Every lit pixel in the image corresponds to a known vector 36-1, 36-2 and 36-3. A point 38-1, 38-2 and 38-3 where this vector 36-1, 36-2 and 36-3, respectively, crosses the surface defined by the laser light 25 is a known point on the surface of the body. If the scanning procedure stops now, a 2-dimensional surface representation of the patient is obtained. However, if a 3-dimensional surface representation is required, several such contour images are used, since each image gives only a single contour 52. If the laser source 20 is translated and/or rotated slightly between each image detection, the imaging device 30 will capture a series of successive contours 52-1, 52-2, 52-3 and 52-4, as illustrated in Fig. 4C. The result of such a laser scan is schematically illustrated in Fig. 4D, where a 3-dimensional representation 60 of a portion of the body surface, contour by contour, is depicted.

This laser scanning technique is generally known as a triangulation technique and its accuracy depends on a number of factors, including resolution of the image detector, accuracy of the laser sweeping mechanism, distance between the scanning device and the image detector, calibration of the scanning device and the image detector with reference to the common coordinate system, width of the laser line and angle at which the laser hits the surface of the body. These parameters are preferably selected and/or optimized before the actual measurements.

Suitable laser scanners applicable with the present invention are commercially available for example from Latronix AB of Sweden. Examples of image detectors that can be used in the triangulation laser scanning above, may be different kinds of cameras, such as CCD (Charged Coupled Device) cameras and CMOS (Complementary Metal Oxide Semiconductor) cameras.

Instead of using triangulation laser scanning, where a sheet of laser light is sent as in Figs. 4A-B, a time-of-flight laser scanning technique may be used. In this technique, a pulsed point laser source sweeps over a portion of patient's body and sends laser light in the form of pulsed laser spots. For a 2-dimensional representation, the laser source sweeps along a determined contour line on the body, whereas for 3-dimensional representations the laser sweeps over one or several predetermined body surface(s). The image detector detects the pulsed laser spots that are reflected off the body surface of the patient. Based on this detected data, using known imaging algorithms, a 2- or 3-dimensional representation of the patient surface is obtained.

A third possible laser scanning technique is an interference-based imaging process. In this technique, the laser beam from the laser source is split into two different beams, a first beam is directed onto the patient, where it is reflected and detected by the image detector, whereas the second beam is directed onto the image detector. In the detector, the patient is depicted as a pattern of light and dark interference bands. This technique has very high-resolution at the cost of complex imaging processing.

Other techniques for determining a 2- or 3-dimensional representation of the patient include ordinary X-ray imaging or even portal imaging. Portal imaging is suited for use in radiation therapy machines, since the same radiation beam as in the treatment procedure may be used. Unfortunately the high energy of the radiation beam gives a fairly poor contrast due to small differences in tissue attenuation at this energy level. Another drawback is the limited effective field of view for most portal imaging system, but if a spatially small patient representation is sufficient, e.g. a representation in the vicinity of standard anatomical reference points, this technique may give a good result. Many conventional portal imagers use a film in physical contact with a metal screen. The metal screen converts the incoming high-energy photons to electrons, which then expose the film resulting in a 2-dimensional image representation of the patient. However, more advanced portal imaging techniques and associated detectors can be used, for an example as suggested by Brahme, et al., in WO 01/59478 A1. By successively rotating the beam source, the obtained 2-dimensional X-ray images may then be combined into a 3-dimensional patient representation.

Also infrared (IR) techniques may be used for determining 2- or 3-dimensional patient representations. In this a case, it is enough to employ an IR detector for detecting the IR radiation irradiating from the patient's body, i.e. in some IR applications no scanning device is required. A rotation of the detector around the patient together with several detector registrations provide a 3-dimensional IR representation of the patient, which may be used according to the present invention.

Several coded aperture imaging techniques are known to the art and may be used for determining the 2- or 3-dimensional representations of the patient according to the present invention. In coded aperture imaging a shift of a mask pattern of incident photon radiation is measured in the shadow created by the mask. From such measurements and adapted software patient representations are obtained.

The above mentioned photon-based techniques are merely given as illustrative examples of techniques applicable to determine the 2- or 3-dimensional representation of the patient and other techniques may also be used in connection with the medical machines according to the invention.

Instead of photons, phonon-imaging or acoustical-imaging may be used by the invention. An acoustical source is then arranged onto or in the vicinity of the medical machines and sends a high-frequent (e.g. ultra sound) acoustical beam onto the patient. A nearby detector is provided for detecting the reflected echo. Based on the detected acoustical data, a representation of the patient is determined according to well-known techniques within the art.

It should also be understood that different patient representation measuring systems may be used in connection with the different medical machines, e.g. a laser scanning system in a first medical machine and a X-ray or portal imaging based system in a second medical machine. However, the laser scanning system determining a surface representation of the patient, discussed above, is preferably arranged at each machine used in the radiation therapy or diagnostic process.

In order to determine the 2- or 3-dimensional representation in the overall coordinate system associated with the patient, preferably a calibration procedure is first performed, which finds a transformation from the coordinate system associated with the patient representation measuring system to the overall coordinate system. A first optional step, is to calibrate the representation measuring system itself. A reference object may be used to adjust the settings of the representation measuring system until a satisfactory representation is obtained, which is well known in the art. Once the representation measuring system is calibrated, its origin and coordinate axes are aligned with the overall common coordinate system. In a preferred embodiment, a reference object is used, the position and orientation of which are determined both in the coordinate system associated with the patient representation measuring system and in the overall coordinate system. A transformation between the two coordinate systems is then obtained, based on the measurements of the reference object. This transformation is subsequently used for all 2- or 3-dimensional representations of that medical machine to get their coordinates in the overall coordinate system. In a radiation therapy or simulation machine, the laser beams used to position the patient based on the isocentric method, as described in the background, may be used to determine the position and orientation of the reference object in the overall coordinate system. If no such laser beams are present, the origin of the overall coordinate system may coincidence with the origin of the 2- or 3-dimenensional representation measuring system, or in the vicinity thereof. If the overall coordinate system coincidence with the coordinate system associated with the representation measuring system, of course no transformation therebetween is required and the calibration procedure described above may be omitted. Another embodiment, where no transformation is required, is if the coordinate system associated with the representation measuring system is used as the overall coordinate system. In such a case, the representation measuring system according to the present invention is preferably arranged onto each diagnostic machine in the diagnostic process and each medical machine (diagnostic and radiation therapy machine) in the radiation therapy process.

In a diagnostic machine according to the invention, anatomical patient information is determined together with the 2- or 3-dimensional representation. A transformation between the local coordinates of the diagnostic machine and the overall coordinate system (preferably based on the transformation between the coordinates associated with the representation measuring system and the overall coordinate system) is determined. A reference object, e.g. one or several balls with a diameter of a couple of centimeters, is placed in the diagnostic machine, where it is depicted both as a 2- or 3-dimensional representation and as anatomical information, using the patient representation measuring system and the diagnostic imaging technique of the machine, respectively. The center of each ball is determined in the local coordinates of the diagnostic machine by calculating their center of gravity. The centers of the balls are also determined based on the 2- or 3-dimensional representation thereof, e.g. by adapting a sphere to the depicted portion of the surfaces of the balls (if a surface representation system is used). A transformation is then obtained from the local coordinates of the diagnostic machine to the corresponding coordinates associated with the patient representation measuring system, and therefore to the overall coordinate system using the transformation determined in the preceding paragraph.

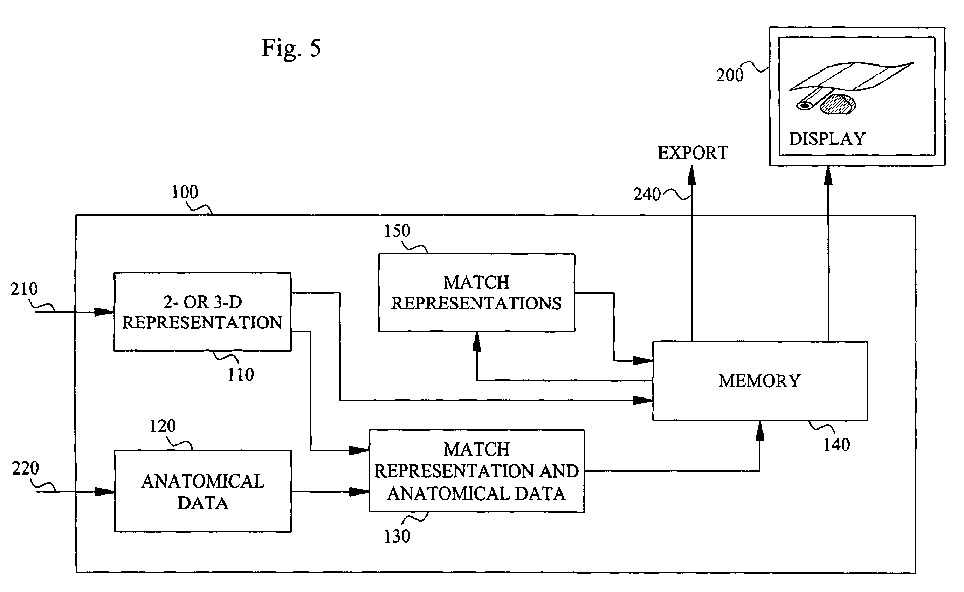

Fig. 5 is a schematic block diagram of a system 100 for relating anatomical patient information between different medical machines according to the invention. Measured imaging data 210 from a 2- or 3-dimensional representation measuring system associated with a first medical machine is input to a processing means 110 in the system 100. The processing means determines the 2- or 3-dimensional patient representation expressed in the common overall coordinate system using the transformation between the coordinate system associated with the representation measuring system and the overall coordinate system, specified above. A corresponding processing means 120 receives diagnostic data 220 and processes it to generate anatomical data of the tumor and relevant tissues and organs. The processed anatomical data is forwarded together with the 2- or 3-dimensional representation recorded in connection with the diagnostic data in the first medical machine to matching means 130 that matches the representation with the anatomical data using the transformation from the local coordinate system of the medical machine and the overall coordinate system. In the matching means 130, the coordinates of the anatomical data are determined in the overall coordinate system based on the transformations. The determined anatomical coordinates are input together with the coordinates for the associated 2- or 3-dimensional representation to a memory 140 in the system 100 and stored.

Imaging data 210 from a second medical machine is input to the system, where processing means 110 determines the coordinates of the 2- or 3-dimensional representation. These coordinates are then stored in the memory 140, which now contains patient representations from two different medical machines and anatomical data associated with the first machine. In order to relate the anatomical data between the different machines, a matching means 150 is configured in the system 100. The matching means 150 determines a conformation that conforms the 2- or 3-dimensional representation of the second medical machine to match the patient representation associated with the first medical machine. Although, both representations are now defined in the common coordinate system, they may have different scales (size), be rotated and/or displaced in relation to each other in the overall coordinate system. The conformation, determined by matching means 150, moves and/or rescale the patient representation of the second medical machine to, as accurately as possible, match or coincidence with the representation of the first machine. The patient representations, of which one may be conformed, are then stored back in the memory 140. The memory 140 may also contain the transformations/conformations determined by the system 100, or they may be stored in the respective means 110, 130 and 150 or provided as input to the system 100.

The data stored in the memory may be displayed on a suitable medium 200, e.g. on a screen or monitor. This screen 200 may of course display both anatomical information from one diagnostic machine alone and/or integrated and coordinated information from several such machines.

The anatomical information, the 2- or 3-dimensional patient representation and their coordinates may also be exported 240 in a suitable format, including the Dicom-format. This exported information 240 may be sent to a computer or data server, stored in whole or part, on or in one or more suitable computer readable media or data storage means such as magnetic disks, CD-ROMs or DVD disks, etc.

The system 100 may be implemented as software, hardware, or a combination thereof. A computer program product implementing the system 100 or a part thereof comprises software or a computer program run on a general purpose or specially adapted computer, processor or microprocessor. The software includes computer program code elements or software code portions illustrated in Fig. 5. The program may be stored in whole or part, on or in one or more suitable computer readable media or data storage means such as magnetic disks, CD-ROMs or DVD disks, hard disks, magneto-optical memory storage means, in RAM or volatile memory, in ROM or flash memory, as firmware, or on a data server. The system 100 may be implemented in a remote computer connected to the medical machines, e.g. arranged in the monitoring room, where the medical personnel are during radiation treatment or diagnostic imaging. A computer arranged onto or in the vicinity of one of the medical machines and connected to the other medical machine(s) may also implement the system 100.

If the two medical machines inputting data into the system 100 are both diagnostic machines, i.e. inputting both imaging data 210 and diagnostic data 220, matching means 130 matches the respective representations with the anatomical data. If no conformation is required, the anatomical information from the two different diagnostic machines can then be stored in the memory 140, exported 240 and/or depicted 200 together in the overall coordinate system. In some applications, however, matching means 150 determines the conformation between the representations in order to meaningfully integrate the anatomical information between the diagnostic machines.

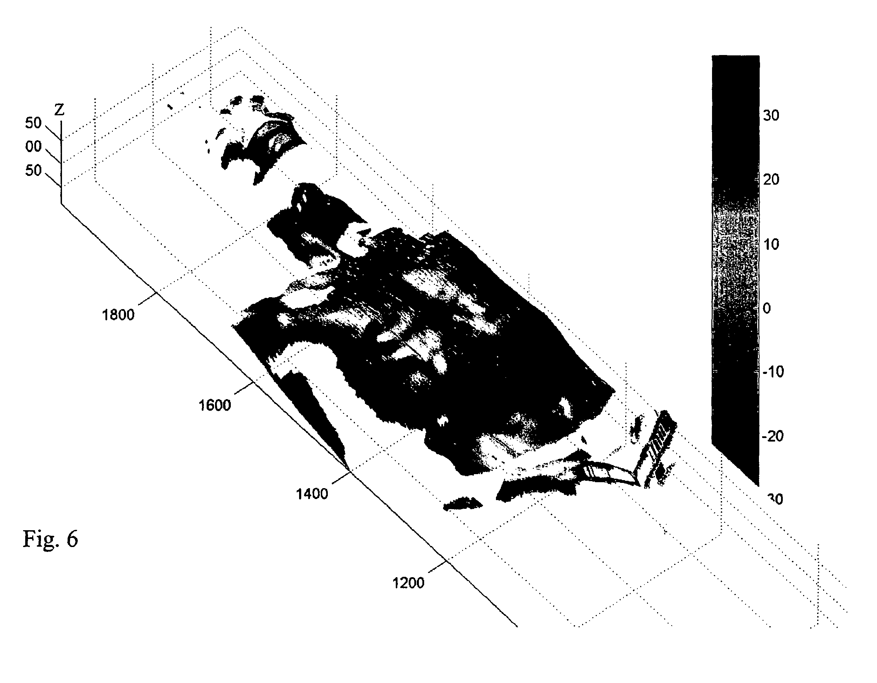

Returning to Fig. 1, the radiation therapy process will briefly be reviewed to discuss possible applications of different embodiments of the present invention in radiation therapy treatment. Starting with obtaining diagnostic data of the tumor and other relevant anatomical tissue. In addition to the ordinary diagnostic data D1, a 2- or 3-dimensional representation of the patient L, e.g. a 3-dimensional surface representation of a portion of the patient's body, is measured and stored in connection with measuring anatomical data D1 in a first diagnostic machine, e.g. a CT diagnostic machine. A reference patient representation is then determined in the overall coordinate system based on this measured 2- or 3-dimensional representation L, using a transformation between the coordinate system associated with the representation measuring system and the overall coordinate system. In order to be able to meaningfully integrate and coordinate anatomical information from different diagnostic machines, it is of importance that the patient is positioned very accurately so that his/hers posture is substantially identical in each machine. As was mentioned in the background, the position and shape of tissues and organs, including the tumor, change depending on the patient's posture. According to the invention, this is solved by first position the patient in an initial position in a second diagnostic machine, e.g. a MR diagnostic machine. Preferably before the actual anatomical measurements, a 2- or 3-dimensional representation of the patient L in the second diagnostic machine is measured and determined in the overall coordinate system. This patient representation is then compared to the reference representation. Since both representations are determined as coordinates in the overall common coordinate system, a deviation between the two representations may be determined. Several different techniques known to the art may be used to determine this deviation. For example, a difference in position of a, preferably every, point on the reference representation and the corresponding point on the patient representation may be expressed as a distance in the coordinate system, possibly together with angles, as a vector or as the distance only along the z-axis. Such a z-axis based measure is schematically depicted in Fig. 6. The gray-scale represents the distance in millimeters between a 3-dimensional reference surface representation and a corresponding representation taken at a subsequent occasion in another machine.

A scalar-based deviation measure is the root mean square (RMS) distance between every point on the reference and patient representation. This gives a simple distance measure that is easily interpreted and may be used to compare the quality of two different matchings.

In order to achieve correct posture and position of the patient, the deviation representation, such as in Fig. 6, may be used to manually reposition the patient. The medical personnel can move, as accurately as possible, the couch, onto which the patient is lying, based on the displayed deviation to a position corresponding to the reference representation. In some cases, it may also or instead be necessary to reposition the patient, i.e. asking him/her to change posture for example by turning the body slightly in some direction. Yet another 2- or 3-dimensional patient representation may then be determined in this new position and compared to the reference presentation as discussed above. Sometimes several such repositions and comparisons are performed before the deviation is below a given threshold value, which is considered accurate enough for radiation therapy purposes.

If the couch is equipped with means for automatic movement of the couch, e.g. motor driven adjusting means, the patient may be positioned automatically. In such a case, a control signal is generated based on the deviation representation. This control signal then causes the adjusting means to move the couch into the correct position, corresponding to a position where the deviation is below a determined threshold. A confirmation of correct position may be carried out, i.e. a new patient representation measurement and comparison, before the actual diagnostic measurement.

Instead of or as a complement to repositioning of the patient couch and/or patient, the diagnostic machine, or more precisely the position of the diagnostic beam source, may be changed relative to the patient. The deviation control signal is then fed into the steering gear of the machine and causes it to reposition the beam source relative the patient in order to reduce the deviation. In this case, the change in position between the diagnostic beam source and the scanning device of the representation measuring system should be accurately known, in order to be able to spatially match the 2- or 3-dimensional representation and the anatomical information.

Once the patient is correctly positioned, anatomical information D2 is measured and stored together with the 2- or 3-dimensional patient representation L, as for the first diagnostic machine. If more anatomical information is needed, further diagnostic machines may be used. The same accurate patient positioning is preferably performed also for these machines.

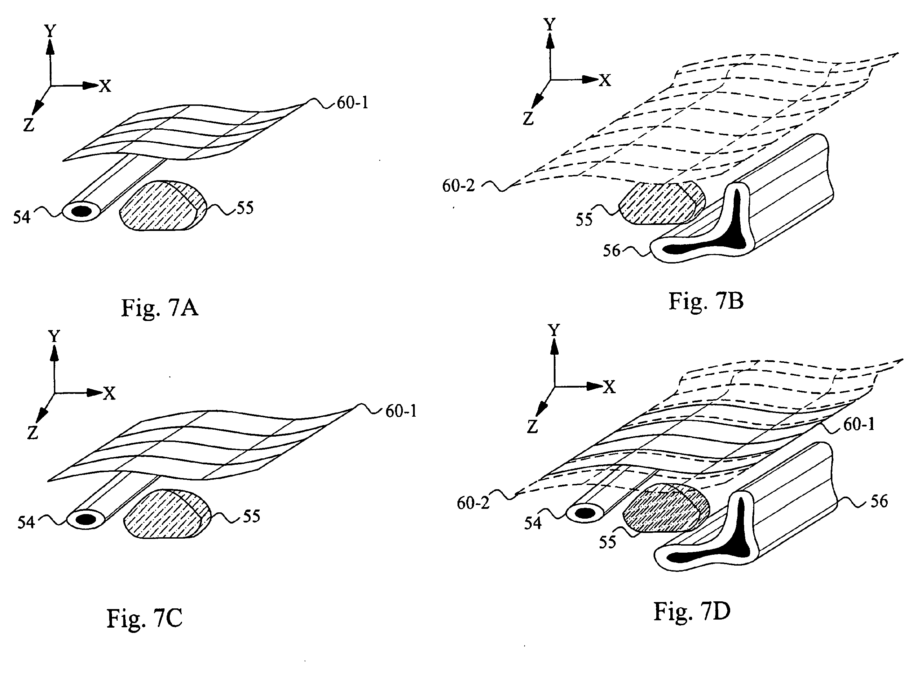

Continuing to the treatment planning, the anatomical information D1, D2, D3 from the different diagnostic machines is coordinated and integrated, e.g. in order to be displayed together in a common picture. Fig. 7A is a schematic illustration of anatomical information 54, 55 from a first diagnostic machine displayed in the common coordinate system together with an associated 3-dimensional surface representation 60-1. In order to display the anatomical information and surface representation, the transformations discussed above have been used, i.e. between the overall coordinate system and the coordinate system associated with the representation measuring system and from the latter coordinate system to the local coordinate system of the diagnostic machine.

A likewise illustration of anatomical information 55, 56 from a second diagnostic machine with an associated surface representation 60-2 is depicted in Fig. 7B, determined using the corresponding transformations.

In order to integrate and coordinate the anatomical information from the different diagnostic machines, the transformations between the local coordinates of the machine and the patient representation measuring system are used. These transformations are combined into a transformation that makes it possible to integrate the anatomical information of the first diagnostic machine to the anatomical information of the second diagnostic machine. All the anatomical information may now be depicted or delineated together in the overall coordinate system. This transformation may, if necessary, incorporate the conformation between the 2- or 3-dimensional representations, discussed above. In such a case, the transformation also considers any differences in scale, position and/or rotation between the patient representations. Such a transformation/conformation, in form of a simple rescale, is illustrated in Fig. 7C.

Once the patient representations 60-1, 60-2 have been matched and possible conformed, the corresponding anatomical information 54, 55, 56 may be integrated and displayed together in the common overall coordinate system, as in Fig. 7D. In such a display, anatomical information 54, 56 obtainable only from specific diagnostic machines may be combined to give a more detailed and comprehensive information of the tumor with adjacent tissues and organs. This display is very accurate and shows relevant anatomical information including mutual spatial relationship between organs and other relevant tissues. This means that organs at risk may be identified and specified relative to the target volume. The information integration and coordination according to the invention is much more accurate than any prior art techniques. In addition, it may be performed completely automatically in a computer. This should be compared to prior used procedures, where the medical personnel manually compare photographs from different medical machines to identify common reference points or tissues.

The resulting comprehensive anatomical information of the invention is the basis for specifying the incidence direction of the treatment radiation beams. In addition, the paths of the beams through the body tissue before hitting the tumor can also be obtained from the information. Based on this information together with information of attenuation and scattering coefficients of different tissues and organs, a correction for the attenuation and scattering of the treatment beams passing through the body to the target volume may be calculated. Therefore, the present invention improves treatment planning markedly by giving better anatomical data, on which the treatment planning is based. As a result, a more accurate treatment plan is obtained. The total time of the treatment planning may also be shortened due to the faster automated data integration.

As a complement to the anatomical data from diagnostic machines, anatomical information from a body or organ atlas may optionally be used in the treatment planning. This atlas is a database or data bank comprising anatomical information of the human body or a portion of it. Such an atlas, may be developed from several different diagnostic measurements collected from different patients. In other words, the atlas is typically a representation of an average human, preferably containing all major organs and tissues, skeleton and nervous system. In order to integrate anatomical data relating to an individual patient, measured by a diagnostic machine according to the invention, with information from the atlas, the associated 2- or 3-dimensional representation of the patient is matched with a corresponding representation in the 'atlas human'. In some cases, since the atlas is an average human, the atlas has to be deformed or conformed to correspond to the actual representation of the patient. Different algorithms may be used to transform the atlas to match the measured representation, e.g. enlarging the 'atlas human' if the present patient is tall. Preferably, such algorithms not only enlarge or reduce the scale of the atlas, but also transform the internal organs and tissue accordingly, based on stored anatomical data in the database. Once the scale of the atlas corresponds to the scale of the measured 2- or 3-dimensional representation, the two are merged or fused so that certain points on the patient representation coincides with corresponding points on the atlas. The measured anatomical information with the tumor may now be displayed together with the organs, tissues and bones of the atlas. From such a combined display, anatomical information, e.g. organs at risk, suitable radiation beam incidence directions, etc., useful in the treatment planning, may be obtained. The atlas may therefore be seen as a complement to diagnostic machines and may be even used to replace some diagnostic machines, reducing the cost and time of the radiation therapy and/or diagnostic process.

The result of the treatment planning is a treatment plan comprising, among other, information on the patient position, irradiation profiles, including incidence direction and irradiation intensities etc., connected to the 2- or 3-dimensional representation of the patient L in the common overall coordinate system.

The treatment plan may then optionally be tested and verified in a simulation. According to the invention, the patient is preferably first positioned in an initial position. Thereafter, a 2- or 3-dimensional representation L is measured and compared with the reference representation in the treatment plan. This may result in a possible deviation or difference representation, e.g. as shown in Fig. 6, based on which a repositioning of the patient is performed as in the case of the diagnostic machine above. Once the patient is accurately positioned, the treatment is simulated, allowing the personnel to make any changes in the treatment plan, such as adding more anatomical information. An actual treatment may then be performed based on the simulation-tested treatment plan.

In the radiation therapy machine, a similar positioning procedure as for the treatment simulation is preferably performed before the irradiation in order to accurately position the patient according to the treatment plan. As was briefly mentioned above, the treatment procedure is often divided into several treatment occasions, possible using different treatment machines T1, T2, T3, which may be distributed over one day, several days, weeks or even months. Preferably before each such treatment occasion, a 2- or 3-dimensional patient representation L is determined, both for accurate positioning purposes but also for detecting any changes in the patient's anatomy. These changes may comprise loss of weight, filling degree of bladder, etc. that all affects the position and shape of internal organs and tissues, including the tumor. For an example, if the patient representation is a 3-dimensional representation of the body surface, a loss of weight is easily detected due to change of the overall shape of the surface representation. In such a case, the treatment plan should be changed to take the new patient anatomy into consideration. This guarantees that an accurate and safe treatment may be accomplished.

However, new 2- or 3-dimensional patient representations L may also be measured and determined during the actual treatment, schematically represented by the dashed boxes in the lower right corner in Fig. 1. These representations may be measured continuously or intermittently at some determined occasions in the treatment procedure. The representations may then e.g. be compared to the reference representation in the treatment plan or to an earlier measured representation in the machine. An important result of such comparisons is that changes in patient position and/or posture during the treatment may be rapidly detected. When the patient is irradiated, it is vital that the patient lies as immovable as possible, since a movement of only a couple of millimeters may cause the radiation beams to hit the target volume in an ineffective direction or even totally miss it. Therefore, the representation measuring and comparison are preferably performed relatively quickly so that any position changes may be detected in 'real time', with a total delay in the order of seconds. If a change in the patient position is detected, a warning signal may be generated if the change exceeds a first threshold value. If the change is large enough to exceed a larger second threshold value, the irradiation is stopped.

If the comparison of the measured representations with the reference is performed after the treatment occasion, the information therefrom may be useful in the follow-up procedure to evaluate the effect of the treatment. In such a case, it is possible to detect any movements and/or misplacement of the patient during the treatment. In the follow-up procedure, the treatment plan may be changed accordingly to adapt for any errors in the positioning. Such a change may include increasing/decreasing the delivered radiation dose in some incident directions to compensate for an earlier too low/high delivered dose, similar to any feed-back changes of the simulation.

Another application of the continuously or intermittently measured patient representations is that movement of the body caused by breathing and/or coughing may be detected. Several organs in the diaphragm are caused to move up and down in connection with the breathing. This breathing associated movement may change the position of the organs 10 to 20 millimeters, with extremes over 30 millimeters between inhalation and exhalation. Continuous patient surface representation measurements may follow such breathing movement by comparing the measured representations with the reference in the treatment plan. Based on these comparisons, the breathing cycle of the patient may be determined and coupled to the organ movement. As a consequence, the dose distribution and the treatment beams may be adapted according to such data. In such a case, the radiation dose is caused to follow the target volume as it is moving with the breathing. This can be accomplished by moving the treatment beam source back and forth correlated to the breathing. Alternatively, the treatment beam may be caused to send only pulsed dose packages, e.g. when the patient is in an exhale or inhale position. This synchronization of beam and breathing gives an increased accuracy of the position of radiation dose relative the target volume.

Continuous or intermittent measurements of the patient representation may also detect the motion of the gantry, depending on the settings of the representation measuring system and the imaging technique it uses. Today, each time the gantry is moved medical personnel have to be present in the treatment room to monitor that there is no collision between the gantry with the radiation head and the couch and patient. This is a tedious and ineffective solution. However, with the present invention the motion of the gantry may effectively and automatically be monitored and any risks of collision may be detected. Thus, no personnel is required in the room during the gantry movement, resulting in a reduction of the total time of the treatment.

The embodiments described above are merely given as examples, and it should be understood that the present invention is not limited thereto. Further modifications, changes and improvements that retain the basic underlying principles disclosed and claimed herein are within the scope of the invention.

## Claims

1. A method for relating anatomical patient information (54-56) between different medical machines (1), said method comprising:
- relating anatomical patient information (54-56) between different medical machines (1) based on 2- or 3-dimensional representations (60; 60-1, 60-2) of at least a portion of a patient (50), **characterized by**:
- determining, in connection with each of the different medical machines (1), a respective 2- or 3-dimensional representation (60; 60-1, 60-2) of at least a portion of the patient (50) using a same type of patient representation measuring system (20, 30) associated with a common coordinate system, wherein said 2- or 3-dimensional patient representations (60; 60-1, 60-2) are employed as common reference between the medical machines (1) when relating the anatomical patient information (54-56).

2. The method according to claim 1, **characterized in that** said step of relating anatomical patient information (54-56) between different medical machines (1) comprises the steps of:
- matching anatomical patient information (54, 55) obtained from a first medical machine with a 2- or 3-dimensional patient representation (60-1) measured in the first medical machine in connection with the generation of the anatomical information (54, 55); and
- matching the 2- or 3-dimensional patient representation (60-1) measured in the first medical machine with a corresponding 2- or 3-dimensional patient representation (60-2) in the second medical machine in order to relate the anatomical patient information (54, 55) obtained from the first medical machine to the second medical machine.

3. The method according to claim 2, **characterized in that** said method further comprises comparing the 2- or 3-dimensional patient representation (60-2) measured in the second medical machine to a reference patient representation based on the 2-or 3-dimensional patient representation (60-1) measured in the first medical machine to enable accurate patient positioning in the second medical machine.

4. The method according to claim 2, **characterized in that** said first and second medical machine (1) are different diagnostic machines and the method further comprises integrating anatomical patient information (55, 56) obtained from the second medical machine with the anatomical patient information (54, 55) from the first medical machine in the common coordinate system using the 2- or 3-dimensional patient representations (60; 60-1, 60-2).

5. The method according to claim 4, **characterized in that** said step of integrating anatomical patient information (54-56) further comprises the steps of:
- transforming a body atlas to match the 2- or 3-dimensional patient representation (60; 60-1, 60-2) measured in at least one of the diagnostic machines; and
- integrating the anatomical patient information (54-56) obtained from the diagnostic machines with standard anatomical information in the body atlas based on the 2- or 3-dimensional patient representation (60; 60-1, 60-2).

6. The method according to any of the claims 1 to 5, **characterized in that** said step of relating anatomical patient information (54-56) between different medical machines (1) comprises determining a respective transformation between the local coordinate system of the different medical machines (1) and the common coordinate system, thereby allowing the anatomical information (54-56) to be integrated together in the common coordinate system.

7. The method according to any of the claims 1 to 6, **characterized in that** said step of relating anatomical patient information (54-56) between different medial machines (1) comprises aligning anatomical patient information (54-56) from different medical machines (1) into the common coordinate system based on the 2- or 3-dimensional representations (60; 60-1, 60-2) of the patient (50).

8. The method according to any of the claims 1 to 7, **characterized in that** one dedicated patient representation measuring system (20, 30) is associated with each of the medical machines (1).

9. The method according to any of the claims 1 to 8, **characterized in that** said 2-or 3-dimensional patient representation (60; 60-1, 60-2) is a 2- or 3-dimensional representation of a predetermined surface of the patient (50).

10. A system (100) for relating anatomical patient information (54-56) between different medical machines (1), said system (100) comprising:
- means (150) for relating anatomical patient information (54-56) between different medical machines (1) based on the 2- or 3-dimensional representations (60; 60-1, 60-2) of at least a portion of a patient (50), **characterized by**:
- means (110) for determining, in connection with each of the different medical machines (1), a respective 2- or 3-dimensional representation (60; 60-1, 60-2) of at least a portion of the patient (50) using a same type of patient representation measuring system (20, 30) associated with a common coordinate system, wherein said relating means (150) employs the 2- or 3-dimensional patient representations as common reference between the medical machines (1).

11. The system according to claim 10, **characterized in that** said means (150) for relating anatomical patient information (54-56) between different medical machines (1) in turn comprises:
- means (130) for matching anatomical patient information (54, 55) obtained from a first medical machine with a 2- or 3-dimensional patient representation (60-1) measured in the first medical machine in connection with the generation of the anatomical information (54, 55); and
- means (150) for matching the 2- or 3-dimensional patient representation (60-1) measured in the first medical machine with a corresponding 2- or 3-dimensional patient representation (60-2) in the second medical machine in order to relate the anatomical patient information (54, 55) obtained from the first medical machine to the second medical machine.

12. The system according to claim 11, **characterized by**:
- means (150) for comparing the 2- or 3-dimensional patient representation (60-2) measured in the second medical machine to a reference patient representation based on the 2- or 3-dimensional patient representation (60-1) measured in the first medical machine and outputting a control signal based on the comparison; and
- positioning means connected to the comparison means (150), adapted for positioning the patient based on the control signal.

13. The system according to claim 11, **characterized in that** said first and second medical machines (1) are different diagnostic machines and said system (100) further comprises means (150) for integrating anatomical patient information (55, 56) obtained from the second medical machine with the anatomical patient information (54, 55) from the first medical machine in the common coordinate system using the 2- or 3-dimensional patient representations (60; 60-1, 60-2).

14. The system according to any of the claims 10 to 13, **characterized in that** said means (150) for relating anatomical patient information (54-56) between different medical machines (1) in turn comprises:
- means (130) for determining a respective transformation between the local coordinate system of the different medical machines (1) and the common coordinate system; and
- means (150) for integrating anatomical information (54-56) together in the common coordinate system based on the transformations received from the means (130) for determining the transformation.

15. The system according to any of the claims 10 to 14, **characterized in that** one dedicated patient representation measuring system (20, 30) is associated with each of the medical machines (1) and said determining means (110) is arranged in the patient representation measuring systems.

16. The system according to any of the claims 10 to 15, **characterized in that** said 2- or 3-dimensional patient representation (60; 60-1, 60-2) is a 2- or 3-dimensional representation of a predetermined surface of the patient (50).

17. The system according to any of the claims 10 to 16, **characterized in that** said patient representation measuring systems (20, 30) comprise:
- photon-emitting means (20) for emitting photon beams (25) onto the patient (50);
- detector (30), arranged to detect photon beams irradiating the patient (50);
- means (110) for determining the 2- or 3-dimensional patient representation (60; 60-1, 60-2) based on the detected photon beams.

## Patentansprüche

1. Verfahren zum in Beziehung setzen von anatomischer Patienteninformation (54-56) zwischen verschiedenen medizinischen Geräten (1), wobei das Verfahren umfasst:
- in Beziehung setzen von anatomischer Patienteninformation (54-56) zwischen verschiedenen medizinischen Geräten (1) basierend auf 2- oder 3-dimensionalen Darstellungen (60; 60-1, 60-2) von zumindest einem Teilbereich eines Patienten (50), **gekennzeichnet durch**
- Bestimmung, in Verbindung mit jedem der verschiedenen medizinischen Geräte (1), einer entsprechenden 2- oder 3-dimensionalen Darstellung (60; 60-1, 60-2) von zumindest einem Teilbereich des Patienten (50) unter Verwendung eines gleichen Typs von mit einem gemeinsamen Koordinatensystem verbundenen Patientendarstellungsmesssystem (20, 30), wobei die 2- oder 3-dimensionalen Patientendarstellungen (60, 60-1, 60-2) als gemeinsame Referenz zwischen den medizinischen Geräten (1) verwendet werden, wenn die anatomische Patienteninformation (54-56) in Beziehung gesetzt wird.

2. Verfahren nach Anspruch 1, **dadurch gekennzeichnet, dass** der Schritt des Verknüpfens anatomischer Patienteninformation (54-56) zwischen verschiedenen medizinischen Geräten (1) die Schritte umfasst:
- Abgleichen durch ein erstes medizinisches Gerät erlangter anatomischer Patienteninformation (54, 55) mit einer in Verbindung mit der Generierung der anatomischen Information (54, 55) in dem ersten medizinischen Gerät gemessenen 2- oder 3-dimensionalen Patientendarstellung (60-1); und
- Abgleichen der in dem ersten medizinischen Gerät gemessenen 2- oder 3-dimensionalen Patientendarstellung (60-1) mit einer entsprechenden 2- oder 3-dimensionalen Patientendarstellung (60-2) in dem zweiten medizinischen Gerät, um die durch das erste medizinische Gerät gewonnene anatomische Patienteninformation (54, 55) mit dem zweiten medizinischen Gerät in Beziehung zu setzen.

3. Verfahren nach Anspruch 2, **dadurch gekennzeichnet, dass** das Verfahren des Weiteren einen Vergleich der in dem zweiten medizinischen Gerät gemessenen 2-oder 3-dimensionalen Patientendarstellung (60-2) mit einer Referenzpatientendarstellung umfasst, die auf der in dem ersten medizinischen Gerät gemessenen 2- oder 3-dimensionalen Patientendarstellung (60-1) basiert, um eine akkurate Patientenpositionierung in dem zweiten medizinischen Gerät zu ermöglichen.

4. Verfahren nach Anspruch 2, **dadurch gekennzeichnet, dass** das erste und zweite medizinische Gerät (1) verschiedene Diagnosegeräte sind und das Verfahren des Weiteren umfasst, dass durch das zweite medizinische Gerät erhaltene anatomische Patienteninformation (55, 56) mit der anatomischen Patienteninformation (54, 55) des ersten medizinischen Geräts in das übliche Koordinatensystem unter Verwendung der 2- oder 3-dimensionalen Patientendarstellungen (60; 60-1, 60-2) integriert wird.

5. Verfahren nach Anspruch 4, **dadurch gekennzeichnet, dass** der Schritt des Integrierens anatomischer Patienteninformation (54,56) des Weiteren die Schritte umfasst:
- Transformieren eines Körperatlasses, um die in zumindest einem der Diagnosegeräte gemessenen 2- oder 3-dimensionalen Patientendarstellungen (60; 60-1, 60-2) abzugleichen, und
- Integrieren der durch die Diagnosegeräte gewonnenen anatomischen Patienteninformation (54-56) mit üblicher anatomischer Information in den Körperatlas basierend auf der 2- oder 3-dimensionalen Darstellung (60; 60-1, 60-2).

6. Verfahren nach einem der Ansprüche 1 bis 5, **dadurch gekennzeichnet, dass** der Schritt des in Beziehung setzens anatomischer Patienteninformation (54-56) zwischen verschiedenen medizinischen Geräten (1) Bestimmung einer entsprechenden Transformation zwischen dem lokalen Koordinatensystem der verschiedenen medizinischen Geräte (1) und dem gemeinsamen Koordinatensystem umfasst, was eine gemeinsame Integration der anatomischen Information (54-56) in das gemeinsame Koordinatensystem ermöglicht.

7. Verfahren nach einem der Ansprüche 1 bis 6, **dadurch gekennzeichnet, dass** der Schritt des in Beziehung setzens anatomischer Patienteninformation (54-56) zwischen verschiedenen medizinischen Geräten (1) Einordnen anatomischer Patienteninformation (54-56) von verschiedenen medizinischen Geräten (1) in das gemeinsame Koordinatensystem basierend auf den 2- oder 3-dimensionalen Darstellungen (60; 60-1, 60-2) des Patienten (50) umfasst.

8. Verfahren nach einem der Ansprüche 1 bis 7, **dadurch gekennzeichnet, dass** ein bestimmtes Patientendarstellungsmesssystem (20, 30) jedem der medizinischen Geräte (1) zugeordnet ist.

9. Verfahren nach einem der Ansprüche 1 bis 8, **dadurch gekennzeichnet, dass** die 2- oder 3-dimensionale Patientendarstellung (60; 60-1, 60-2) eine 2- oder 3-dimensionale Darstellung einer vorbestimmten Fläche des Patienten (50) ist.

10. System (100) zum in Beziehung setzen anatomischer Patienteninformation (54-56) zwischen verschiedenen medizinischen Geräten (1), wobei das System (100) umfasst:
- Mittel (150) zum in Beziehung setzen anatomischer Patienteninformation (54-56) zwischen verschiedenen medizinischen Geräten (1) basierend auf den 2- oder 3-dimensionalen Darstellungen (60; 60-1, 60-2) von zumindest einem Teilbereich eines Patienten (50), **gekennzeichnet durch**:
- Mittel (110) zur Bestimmung, in Verbindung mit jedem der verschiedenen medizinischen Geräte (1), einer entsprechenden 2- oder 3-dimensionalen Darstellung (60; 60-1, 60-2) von zumindest einem Teilbereich des Patienten (50) unter Verwendung eines gleichen Typs von mit einem gemeinsamen Koordinatensystem verbundenen Patientendarstellungsmesssystem (20, 30), wobei das Mittel (150) zum in Beziehung setzen die 2- oder 3-dimensionalen Patientendarstellungen als gemeinsame Referenz zwischen den medizinischen Geräten (1) verwendet.

11. System nach Anspruch 10, **dadurch gekennzeichnet, dass** das Mittel (150) zum in Beziehung setzen anatomischer Patienteninformation (54-56) zwischen verschiedenen medizinischen Geräten (1) wiederum umfasst:
- Mittel (130) zum Abgleichen durch ein erstes medizinisches Gerät erlangter anatomischer Patienteninformation (54, 55) mit einer in Verbindung mit der Generierung der anatomischen Information (54, 55) in dem ersten medizinischen Gerät gemessenen 2- oder 3-dimensionalen Patientendarstellung (60-1); und
- Mittel (150) zum Abgleichen der in dem ersten medizinischen Gerät gemessenen 2- oder 3-dimensionalen Patientendarstellung (60-1) mit einer entsprechenden 2- oder 3-dimensionalen Patientendarstellung (60-2) in dem zweiten medizinischen Gerät, um die durch das erste medizinische Gerät gewonnene anatomische Patienteninformation (54, 55) mit dem zweiten medizinischen Gerät in Beziehung zu setzen.

12. System nach Anspruch 11, **gekennzeichnet durch**:
- Mittel (150) zum Vergleich der in dem zweiten medizinischen Gerät gemessenen 2- oder 3-dimensionalen Patientendarstellung (60-2) mit einer Referenzpatientendarstellung, die auf der in dem ersten medizinischen Gerät gemessenen 2- oder 3-dimensionalen Patientendarstellung (60-1) basiert, und zur Ausgabe eines auf dem Vergleich basierenden Kontrollsignals; und
- mit dem Vergleichsmittel (150) verbundenes Positionierungsmittel, das geeignet ist, um den Patienten basierend auf dem Kontrollsignal zu positionieren.

13. System nach Anspruch 11, **dadurch gekennzeichnet, dass** das erste und zweite medizinische Gerät (1) verschiedene Diagnosegeräte sind und das System (100) des Weiteren Mittel (150) umfasst zur Integration von durch das zweite medizinische Gerät erhaltener anatomischer Patienteninformation (55, 56) mit der anatomischen Patienteninformation (54, 55) des ersten medizinischen Geräts in das gemeinsame Koordinatensystem unter Verwendung der 2- oder 3-dimensionalen Patientendarstellungen (60; 60-1, 60-2).

14. System nach einem der Ansprüche 10 bis 13, **dadurch gekennzeichnet, dass** das Mittel (150) zum in Beziehung setzen anatomischer Patienteninformation (54-56) zwischen verschiedenen medizinischen Geräten (1) wiederum umfasst:
- Mittel (130) zur Bestimmung einer entsprechenden Transformation zwischen dem lokalen Koordinatensystem der verschiedenen medizinischen Geräte (1) und dem gemeinsamen Koordinatensystem; und
- Mittel (150) zur gemeinsamen Integration anatomischer Information (54-56) in das gemeinsame Koordinatensystem basierend auf den Transformationen, die von dem Mittel (130) zur Bestimmung der Transformation erhalten wurden.

15. System nach einem der Ansprüche 10 bis 14, **dadurch gekennzeichnet, dass** ein bestimmtes Patientendarstellungsmesssystem (20, 30) jedem der medizinischen Geräte (1) zugeordnet ist und das Bestimmungsmittel (110) in dem Patientendarstellungsmesssystem angeordnet ist.

16. System nach einem der Ansprüche 10 bis 15, **dadurch gekennzeichnet, dass** die 2- oder 3-dimensionale Patientendarstellung (60; 60-1, 60-2) eine 2- oder 3-dimensionale Darstellung einer vorbestimmten Fläche des Patienten (50) ist.

17. System nach einem der Ansprüche 10 bis 16, **dadurch gekennzeichnet, dass** die Patientendarstellungsmesssysteme (20, 30) umfassen:
- photonenemittierendes Mittel (20), um Photonenstrahlen (25) auf den Patienten (50) zu emittieren;
- Detektor (30), angeordnet, um Photonenstrahlen zu erfassen, die den Patienten (50) bestrahlen;
- Mittel (110) zur Bestimmung der 2- oder 3-dimensionalen Patientendarstellung (60; 60-1, 60-2) basierend auf den erfassten Photonenstrahlen.

## Revendications

1. Procédé d'associations d'informations anatomiques d'un patient (54-56) entre différentes machines médicales (1), ledit procédé comprenant :
- l'association d'informations anatomiques d'un patient (54-56) entre différentes machines médicales (1) basées sur des représentations bi- ou tridimensionnelles (60 ; 60-1, 60-2) d'au moins une partie d'un patient (50), **caractérisé** pat :
- la détermination, en relation avec chacune des différentes machines médicales (1), d'une représentation bi- ou tridimensionnelle respective (60 ; 60-1, 60-2) d'au moins une partie du patient (50) en utilisant un même type de système de mesure de représentation d'un patient (20, 30) associée à un système de coordonnées commun, dans lequel lesdites représentations bi- ou tridimensionnelles du patient (60 ; 60-1, 60-2) sont utilisées comme référence commune entre les machines médicales (1) lorsque l'on associe les informations anatomiques du patient (54-56).

2. Procédé selon la revendication 1, **caractérisé en ce que** ladite étape d'association des informations anatomiques du patient (54-56) entre différentes machines médicales (1) comprend les étapes consistant à :
- apparier des informations anatomiques du patient (54, 55) obtenues à partir d'une première machine médicale avec une représentation bi- ou tridimensionnelle du patient (60-1) mesurée dans la première machine médicale en rapport avec la génération des informations anatomiques (54, 55) ; et
- apparier la représentation bi- ou tridimensionnelle du patient (60-1) mesurée dans la première machine médicale avec une représentation bi- ou tridimensionnelle correspondante du patient (60-2) dans la deuxième machine médicale afin d'associer les informations anatomiques du patient (54, 55) obtenues de la première machine médicale à la deuxième machine médicale.

3. Procédé selon la revendication 2, **caractérisé en ce que** ledit procédé comprend en outre la comparaison de la représentation bi- ou tridimensionnelle du patient (60-2) mesurée dans la deuxième machine médicale à une représentation de référence du patient basée sur la représentation bi- ou tridimensionnelle du patient (60-1) mesurée dans la première machine médicale pour permettre le placement précis du patient dans la deuxième machine médicale.

4. Procédé selon la revendication 2, **caractérisé en ce que** lesdites première et deuxième machines médicales (1) sont des machines de diagnostic différentes et le procédé comprend en outre l'intégration des informations anatomiques du patient (55, 56) obtenues de la deuxième machine médicale avec les informations anatomiques du patient (54, 55) de la première machine médicale dans le système de coordonnées commun en utilisant les représentations bi- ou tridimensionnelles du patient (60 ; 60-1, 60-2).

5. Procédé selon la revendication 4, **caractérisé en ce que** ladite étape d'intégration des informations anatomiques du patient (54-56) comprend en outre les étapes consistant à :
- transformer un atlas corporel pour apparier les représentations bi- ou tridimensionnelles du patient (60 ; 60-1, 60-2) mesurées dans au moins l'une des machines de diagnostic ; et
- intégrer les informations anatomiques du patient (54-56) obtenues des machines de diagnostic avec des informations anatomiques standard dans l'atlas corporel basées sur la représentation bi- ou tridimensionnelle du patient (60 ; 60-1, 60-2).

6. Procédé selon l'une quelconque des revendications 1 à 5, **caractérisé en ce que** ladite étape d'association des informations anatomiques du patient (54-56) entre différentes machines médicales (1) comprend la détermination d'une transformation respective entre le système de coordonnées local des différentes machines médicales (1) et le système de coordonnées commun, permettant ainsi l'intégration des informations anatomiques (54-56) conjointement dans le système de coordonnées commun.

7. Procédé selon l'une quelconque des revendications 1 à 6, **caractérisé en ce que** ladite étape d'association des informations anatomiques du patient (54-56) entre différentes machines médicales (1) comprend l'alignement d'informations anatomiques du patient (54-56) de différentes machines médicales (1) dans le système de coordonnées commun basées sur les représentations bi- ou tridimensionnelles (60 ; 60-1, 60-2) du patient (50).

8. Procédé selon l'une quelconque des revendications 1 à 7, **caractérisé en ce qu'**un système de mesure de représentation spécifique du patient (20, 30) est associé à chacune des machines médicales (1).

9. Procédé selon l'une quelconque des revendications 1 à 8, **caractérisé en ce que** ladite représentation bi- ou tridimensionnelle du patient (60 ; 60-1, 60-2) est une représentation bi- ou tridimensionnelle d'une surface prédéterminée du patient (50).

10. Système (100) permettant d'associer des informations anatomiques d'un patient (54-56) entre différentes machines médicales (1), ledit système (100) comprenant :
- un moyen (150) pour associer des informations anatomiques du patient (54-56) entre différentes machines médicales (1) basées sur les représentations bi- ou tridimensionnelles (60 ; 60-1, 60-2) d'au moins une partie d'un patient (50), **caractérisé par :**
- un moyen (110) de détermination, en relation avec chacune des différentes machines médicales (1), d'une représentation bi- ou tridimensionnelle respective (60 ; 60-1, 60-2) d'au moins une partie du patient (50) en utilisant un même type de système de mesure de représentation d'un patient (20, 30) associé à un système de coordonnées commun, dans lequel ledit moyen d'association (150) utilise les représentations bi- ou tridimensionnelles comme référence commune entre les machines médicales (1).

11. Système selon la revendication 10, **caractérisé en ce que** ledit moyen (150) d'association d'informations anatomiques du patient (54-56) entre différentes machines médicales (1) comprend lui-même :
- un moyen (130) d'appariement d'informations anatomiques du patient (54, 55) obtenues d'une première machine médicale avec une représentation bi- ou tridimensionnelle du patient (60-1) mesurées dans la première machine médicale en relation avec la génération des informations anatomiques (54, 55) ; et
- un moyen (150) d'appariement des représentations bi- ou tridimensionnelles du patient (60-1) mesurées dans la première machine médicale avec une représentation bi- ou tridimensionnelle correspondante du patient (60-2) dans la deuxième machine médicale afin d'associer les informations anatomiques du patient (54, 55) obtenues de la première machine médicale à la deuxième machine médicale.

12. Système selon la revendication 11, **caractérisé par** :
- un moyen (150) de comparaison des représentations bi- ou tridimensionnelles du patient (60-2) mesurées dans la deuxième machine médicale à une représentation de référence du patient basée sur les représentations bi- ou tridimensionnelles du patient (60-1) mesurées dans la première machine médicale et l'émission d'un signal témoin sur la base de la comparaison ; et
- un moyen de positionnement relié au moyen de comparaison (150) adapté pour positionner le patient sur la base du signal témoin.

13. Système selon la revendication 11, **caractérisé en ce que** lesdites première et deuxième machines médicales (1) sont des machines de diagnostic différentes et ledit système (100) comprend en outre un moyen (150) d'intégration d'informations anatomiques du patient (55, 56) obtenues de la deuxième machine avec les informations anatomiques du patient (54, 55) de la première machine médicale dans le système de coordonnées commun en utilisant les représentations bi- ou tridimensionnelles du patient (60 ; 60-1, 60-2).

14. Système selon l'une quelconque des revendications 10 à 13, **caractérisé en ce que** ledit moyen (150) d'association d'informations anatomiques du patient (54-56) entre différentes machines médicales (1) comprend lui-même :
- un moyen (130) de détermination d'une transformation respective entre le système de coordonnées local des différentes machines médicales (1) et le système de coordonnées commun ; et
- un moyen (150) d'intégration d'informations anatomiques (54-56) conjointement dans le système de coordonnées commun basées sur les transformations reçues du moyen (130) de détermination de la transformation.

15. Système selon l'une quelconque des revendications 10 à 14, **caractérisé en ce qu'**un système de mesure de représentation spécifique du patient (20, 30) est associé à chacune des machines médicales (1) et ledit moyen de détermination (110) est disposé dans les systèmes de mesure de représentation du patient.

16. Système selon l'une quelconque des revendications 10 à 15, **caractérisé en ce que** lesdites représentations bi- ou tridimensionnelles du patient (60 ; 60-1, 60-2) sont une représentation bi- ou tridimensionnelle d'une surface prédéterminée du patient (50).

17. Système selon l'une quelconque des revendications 10 à 16, **caractérisé en ce que** lesdits systèmes de mesure de représentation du patient (20, 30) comprennent :
- un moyen d'émission de photons (20) pour émettre des faisceaux de photons (25) sur le patient (50) ;
- un détecteur (30) conçu pour détecter des faisceaux de photons irradiant le patient (50) ;
- un moyen (110) de détermination de la représentation bi- ou tridimensionnelle du patient (60 ; 60-1, 60-2) basée sur les faisceaux de photons détectés.
